(19) Europäisches Patentamt
European Patent Office
Office européen des brevets

(11) **EP 4 153 668 B1**

(12) **EUROPÄISCHE PATENTSCHRIFT**

(45) Veröffentlichungstag und Bekanntmachung des Hinweises auf die Patenterteilung:
**28.08.2024 Patentblatt 2024/35**

(21) Anmeldenummer: **21725199.0**

(22) Anmeldetag: **17.05.2021**

(51) Internationale Patentklassifikation (IPC):
***C08J 9/00*** *(2006.01)* ***C07C 233/43*** *(2006.01)*
***C07C 233/44*** *(2006.01)* ***C08J 9/12*** *(2006.01)*
***C08J 9/14*** *(2006.01)* ***C08K 5/00*** *(2006.01)*
***C08K 5/20*** *(2006.01)*

(52) Gemeinsame Patentklassifikation (CPC):
(C-Sets verfügbar)
**C08J 9/0028; C07C 233/43; C07C 233/62; C07C 233/80; C08J 9/122; C08J 9/141; C08K 5/20;**
C07C 2601/14; C08J 2201/024; C08J 2203/06; C08J 2203/14; C08J 2205/044; C08J 2205/052; C08J 2325/06; C08J 2325/12 (Forts.)

(86) Internationale Anmeldenummer:
**PCT/EP2021/062951**

(87) Internationale Veröffentlichungsnummer:
**WO 2021/233811 (25.11.2021 Gazette 2021/47)**

(54) **KLEINZELLIGE POLYSTYROL-SCHÄUME UND VERFAHREN ZU DEREN HERSTELLUNG**

SMALL-CELL POLYSTYRENE FOAMS, AND PROCESS FOR PRODUCING SAME

MOUSSES DE POLYSTYRÈNE À PETITES CELLULES ET LEUR PROCÉDÉ DE PRODUCTION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.05.2020 EP 20175406**

(43) Veröffentlichungstag der Anmeldung:
**29.03.2023 Patentblatt 2023/13**

(73) Patentinhaber: **INEOS Styrolution Group GmbH**
**60325 Frankfurt (DE)**

(72) Erfinder:
- **SCHMIDT, Hans-Werner**
**95444 Bayreuth (DE)**
- **KREGER, Klaus**
**95676 Wiesau (DE)**
- **KLOSE, Bastian**
**95444 Bayreuth (DE)**

(74) Vertreter: **Jacobi, Markus Alexander**
**Patentanwälte**
**Isenbruck Bösl Hörschler PartG mbB**
**Eastsite One**
**Seckenheimer Landstrasse 4**
**68163 Mannheim (DE)**

(56) Entgegenhaltungen:
**EP-A2- 1 031 600** **WO-A1-2015/090509**
**DE-A1- 102011 083 434** **US-B2- 8 420 721**

- **KLOSE BASTIAN ET AL: "Kinked Bisamides as Efficient Supramolecular Foam Cell Nucleating Agents for Low-Density Polystyrene Foams with Homogeneous Microcellular Morphology", POLYMERS, vol. 13, no. 7, 30 March 2021 (2021-03-30), CH, pages 1094, XP055822502, ISSN: 2073-4360, DOI: 10.3390/polym13071094**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**C08K 5/20, C08L 25/12**

## Beschreibung

Die vorliegende Erfindung betrifft ein Verfahren zur Herstellung von kleinzelligen Schaummaterialien aus wenigstens einem Styrol-haltigen polymeren Material. Gegenstand sind auch nach diesem Verfahren hergestellte Schaumstoffe sowie die Verwendung von bestimmten aromatischen Carbonsäureamiden als zellverkleinernde Additive in Schaumstoffen. Die Polymerschäume sollen dabei vorzugsweise geschlossen-zellig sein. Auch soll die mittlere Zellgröße der Schäume klein sein und vorzugsweise nicht stark variieren.

Als "offenzelliger Polymerschaum" wird im Gegensatz zum geschlossen-zelligen Schaum ein Polymerschaum verstanden, der wenigstens 20% offene Zellen (bezogen auf Anzahl der Zellen), bestimmt nach der Norm ASTM-D 2856-A (Open-Cell Content in Cellular Plastics), aufweist.

Verschiedenen Verfahren zur Herstellung von Schaumstoffen basierend auf Polymeren und organischen Hilfskomponenten sind aus dem Stand der Technik bekannt. Verschiedene Nukleierungsmittel werden als Additive in der Literatur, insbesondere Kristallnukleierung und zur Erhöhung der Transparenz, in teilkristallinen Polymeren zugesetzt.

Überraschend hat es sich gezeigt, dass die Verwendung von, in thermoplastischen, styrolhaltigen Polymeren (in der Schmelze) gut löslichen, organischen gewinkelten Carbonsäure-bisamiden die Herstellung von geschlossenzelligem Polymerschaum mit kleiner Zellengröße ermöglicht. Das erfindungsgemäße Verfahren lässt sich effizient und kostengünstig durchführen, sowohl im Kleinen wie auch im industriellen Maßstab. Damit können unter Einsatz von Styrol-Polymeren und diesen Additiven geschlossen-zellige Polymerschäume mit sehr kleiner mittlerer Zellgröße hergestellt werden, wobei diese Schäume insbesondere eine nahezu monomodale Zellgrößenverteilung haben.

Im Stand der Technik beschreibt z.B. EP-A 681522 (Dow, 1994) ein Verfahren zur Herstellung eines Polystyrol-Schaumstoffs mit geschlossenen Zellen, bei dem eine Schmelze mit einem Zellvergrößerungs-Agens und einem Treibmittel versetzt wird und die erhaltene schäumbare Mischung bei niedrigerem Druck zum Schaumstoff verarbeitet wird, als Additiv wird z.B. ein Polyethylen-Glykol eingesetzt.

US 5210105 (Dow, 1993) offenbart die Herstellung von Polystyrol-Schaum mit einer mittleren Zellengröße von 50-1200 $\mu$m.

EP-A 1031600 (BASF, 2000) lehrt die Herstellung von dicken Schaumstoffplatten aus Styrol-Polymeren mit verminderter Wärmeleitfähigkeit, die Kohlenstoffpartikel enthalten. Die Platten werden zum Isolieren eingesetzt und haben eine mittlere Zellenzahl von z.B. 4-9, sie enthalten z.B. 1% Graphit.

EP-B 1385902 (BASF, 2009) offenbart ein Verfahren zur Herstellung von Schaumstoffen durch Extrudieren und Aufschäumen einer Mischung aus Styrol-Polymer, 3 bis 15 Gew.-% Treibmittelgemisch und 0,01 bis 10 Gew.-% Graphitpartikeln, bei dem als Treibmittel ein Gemisch aus Kohlenstoffdioxid, Ethanol, einem aliphatischen $C_3$-$C_5$-Kohlenwasserstoff und Wasser verwendet wird.

WO 2004/072168 (Ciba) beschreibt die Herstellung von verschiedenen Tris-Carbonsäureamiden, die in Polypropylen Produkten als Additive Verwendung finden.

EP-A 1661939 (BASF, 2006) beschreibt Schaumstoffe auf Basis von Styrol-Polymeren mit einer Dichte im Bereich von 20 bis 200 kg/$m^3$, einer mittleren Zellgröße von 0,08 bis 0,25 mm und einer mittleren Zellwanddicke von 350 bis 1500 nm. Es werden Platten mit verminderter Wärmeleitfähigkeit und hoher Druckfestigkeit hergestellt, die Verwendung von speziellen Additiven zur Zellverkleinerung wird nicht offenbart.

Die Publikation von M. Stumpf et al. in Journal of Cellular Plastics 2011, 47(6), 519-534 lehrt die Herstellung von verschiedenen isotaktischen Polypropylen-Schäumen unter Einsatz von Benzol-Trisamiden als Schaum-Nukleierungsmittel.

US 8,420,721 (Adeka, 2013) offenbart die Synthese von verschiedenen aromatischen Bisamid-Verbindungen, die zusammen mit weiteren Additiven in Polyolefinen (PE/PP) verwendet werden. DE-A 102011083434 (NMB, 2013) beschreibt ein Verfahren zur Herstellung von offenzelligen Polymer-Schäumen unter Verwendung von aromatischen Trisamid-Verbindungen und langkettenverzweigtem HMS-Polypropylen (Daploy WB 140 HMS, Borealis). Als Nukleierungsmittel werden 1,3,5-Tris(2,2-dimethylpropionyl-amino)benzol und Tris(1,1,3,3-tetramethylbutyl)-1,3,5-benzol-tricarboxamid eingesetzt, die Herstellung erfolgt über die Schritte: Herstellen der Polymerschmelze, Lösen des organischen Nukleierungsmittels in der Schmelze, Versetzen der Schmelze mit Treibmittel und Extrudieren der Schmelze.

WO 2015/090509 und EP-B 3083802 (Clariant) beschreiben den Einsatz von aromatischen Trisamid-Verbindungen zur Herstellung von geschäumten Polymerartikeln, die z.B. eine Dichte von 10-65 kg/$m^3$ aufweisen können. Als bevorzugtes Nukleierungs-Additiv wird 1,3,5-Tris(2,2-dimethyl-proprionyl-amino)-benzol (Irgaclear® XT 386; BASF, Ludwigshafen, Formel (V)) der nachfolgenden Formel eingesetzt. Dieses führt jedoch nicht zu einheitlich kleinen Zellgrößen im Polymerschaum.

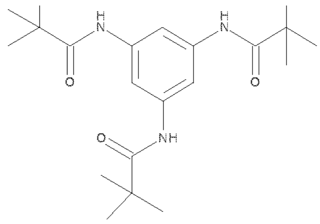

(V)

WO 2015/197152 (Clariant) lehrt die Herstellung von Polymer-Schäumen, z.B. Polystyrol-Schaum basierend auf Polystyrol PS153F (INEOS Styrolution, Frankfurt,), unter Verwendung einer löslichen Benzyliden-Sorbitol-Verbindung der allgemeinen Formel (VI) als Keimbildner bei der Herstellung.

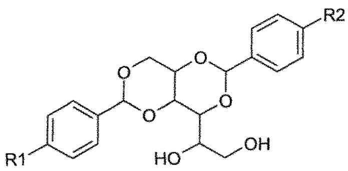

(VI)

Der Artikel von M. Mörl et al. in Journal of Cellular Plastics 2018, 54, 483-498 beschreibt die Herstellung von Polypropylen-Schäumen mit mittelgroßen Zellen, basierend auf Moplen HF400G (Lyondell-Basell) und verschiedenen aromatischen Trisamidbasierten Nukleierungsmitteln.

Der Artikel von M. Aksit und B. Klose et al. in Journal of Cellular Plastics 2019, 55(3), 249 - 261 lehrt die Herstellung von Polystyrol-Schaum basierend auf Polystyrol PS168N (INEOS Styrolution, Frankfurt) unter Einsatz von 1,3,5-Tris(2,2-dimethyl-propionylamino)-benzol (Irgaclear XT386, BASF). Die Dichte des PS-Schaums wurde über die Wasserverdrängung (ISO 1183) bestimmt, die Struktur über Elektronenmikroskopie (REM). Einheitlich kleine Zellgrößen im Polymerschaum werden nicht gelehrt.

Die Veröffentlichung von M. Aksit, C. Zhao et al. in Polymers, 2019, 11, 268ff. beschreibt die Herstellung von Polystyrol-Schaum unter Einsatz von Benzol-Trisamid-Verbindungen wie 1,3,5-Tris(2,2-dimethyl-proprionylamino)-benzol (Irgaclear XT386, BASF) und erwähnt eine Reduktion der Wärmeleitfähigkeit der Schäume.

Selbst bei Verwendung von 0,5 Gew.-% des Nukleierungs-Additivs hat der Schaum eine Zellengröße von über 30 Mikrometer.

Im Stand der Technik wird auch der Zusatz von Graphit oder Talkum für die Herstellung von Schaumstoffen aus polymeren Materialien vorgeschlagen, der Zusatz dieser stark nukleierenden Additive bewirkt bei der Herstellung des Schaumstoffs kleinere Zellen, eingesetzt in Mengen über 0,2 Gew.-%, bezogen auf das polymere Material. Diese Additive können sich jedoch negativ auf das Expansionsverhalten des Schaums auswirken.

Im Rahmen der vorliegenden Erfindung spielt die Dichte des Schaumstoffs eine Rolle, auch der mittlere Zellendurchmesser (D) des Schaumstoffs (in Mikrometer) und die Struktur des Schaums (geschlossen-zellig) sowie die Morphologie der Zellen sind wichtig. Auch die Homogenität der Zellen spielt eine wichtige Rolle, stark uneinheitliche Zellen größen sind für viele Anwendungen von Nachteil.

Als Maß für die Größe der Zellen kann der mittlere Zellendurchmesser aber auch "Zellen pro Millimeter" (in jeder der drei Raumrichtungen pro Millimeter Strecke) für die erfindungsgemäß hergestellten Schaumstoffe angegeben werden. Ein Zellenzahl-Wert von 200 Zellen pro mm beschreibt z.B. einen sehr kleinzelligen Schaum, ein Wert von 8 z.B. einen großzelligen Schaum.

Eine Aufgabe der vorliegenden Erfindung ist es, ein einfaches Verfahren zur Herstellung von kleinzelligen Schaumstoffen mit (weitgehend) geschlossenen Zellen ausgehend von styrolhaltigen Polymeren und von kostengünstig herstellbaren Additiven bereitzustellen. Die Größe und Struktur der Schaumzellen kann durch die später beschriebenen Verfahren untersucht werden.

Diese Aufgabe wird gelöst durch ein Verfahren zur Herstellung eines Schaumstoffs aus mindestens einer Styrol-Polymer-Komponente (S) und mindestens einem Additiv der allgemeinen Formel (I) und das Verfahren umfasst die Schritte:

a. Erwärmen mindestens einer Styrol-Polymer-Komponente (S), um eine geschmolzene, polymere Formmasse zu erhalten,

b. Einbringen eines Treibmittels (T) in die geschmolzene, polymere Formmasse zur Ausbildung einer schäumbaren Zusammensetzung (Z), und

c. Aufschäumen der schäumbaren Zusammensetzung (Z), um einen geschäumten Formkörper zu erhalten,

wobei in der geschmolzenen polymeren Formmasse mindestens ein Carbonsäureamid der allgemeinen Formel (I) eingesetzt wird,

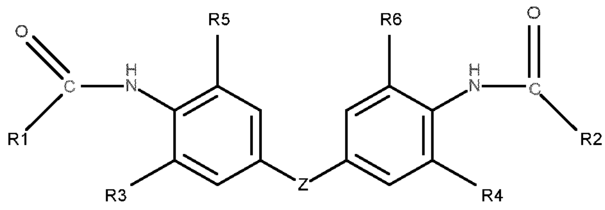

(I)

wobei bedeuten:

Z eine $C_1$-$C_5$-, insbesondere $C_1$-$C_3$-Alkylengruppe, oftmals -$CH_2$-, oder ein Sauerstoffatom oder Schwefelatom, oftmals ein Sauerstoffatom;
R1 und R2 unabhängig voneinander einen verzweigten $C_3$-$C_{12}$-Alkylrest oder unverzweigten $C_1$-$C_{12}$-Alkylrest, einen $C_3$-$C_{12}$-Cycloalkylrest, oder einen Benzylrest;
R3, R4, R5 und R6 jeweils unabhängig voneinander Wasserstoff, einen unverzweigten $C_1$-$C_6$-Alkylrest oder einen verzweigten $C_3$-$C_6$-Alkylrest.

Beim Verfahren zur Herstellung eines Schaumstoffs aus Styrol-Polymer-Komponente (S) und Additiv der Formel (I) kann es sich um verschiedene typische Verfahren zur Herstellung von Polymerschäumen handeln.

Es kann ein einfaches "Batchschaum-Verfahren" (im kleinen Maßstab) sein, bei dem ein Treibmittel (T) über eine Pumpe in einen Autoklaven mit der Polymerzusammensetzung eingebracht wird.

Es kann sich aber auch um ein (z.B. industrielles) Schaum-Extrusions-Verfahren handeln, bei dem z.B. ein Polystyrol (und/oder ein Styrolcopolymer) und das bzw. die Additive in einen Extruder (z.B. Doppelschneckenextruder) eingebracht und erhitzt werden (z.B. 260°C), dann eine Injektion des Treibmittels erfolgt, um die Zusammensetzung dann ggf. über einen weiteren Extruder (mit geringerer Temperatur) der Schaumbildungs-Einheit zuzuführen.

Die als Additiv eingesetzten "gewinkelten" aromatischen Carbonsäureamid-Verbindungen (Derivate) sind Bisamide und weisen vorzugsweise als Gruppe Z eine Methylengruppe auf. Sie sind vorzugsweise in den eingesetzten Polymeren, insbesondere bei der Verarbeitungstemperatur, gut löslich.

In einer Ausführungsform der Erfindung bedeuten in der allgemeinen Formel (I) R1 und R2 unabhängig voneinander einen $C_3$-$C_{12}$-Cycloalkylrest, insbesondere einen Cyclohexylrest oder Cyclopentylrest, einen Butylrest oder einen Benzylrest.

In einer Ausführungsform der Erfindung bedeuten in der allgemeinen Formel (I) Z eine Methylengruppe, und R3, R4, R5 und R6 jeweils unabhängig voneinander einen $C_1$-$C_6$-Alkylrest, insbesondere einen $C_1$-$C_2$-Alkylrest.

In einer Ausführungsform der Erfindung bedeuten in Formel (I) Z eine -$CH_2$-Gruppe oder ein Sauerstoffatom; R1 und R2 unabhängig voneinander einen verzweigten oder unverzweigten $C_4$-$C_6$-Alkylrest, einen $C_5$-$C_6$-Cycloalkylrest, oder einen Benzylrest; R3, R4, R5 und R6 jeweils unabhängig voneinander Wasserstoff oder einen unverzweigten $C_1$-$C_3$-Alkylrest, insbesondere einen Methyl- oder Ethylrest.

In einer Ausführungsform der Erfindung wird im Verfahren das Carbonsäure-bisamid-Derivat der allgemeinen Formel (I) in einer Menge von 0,01 bis 2,0 Gew.-%, insbesondere 0,05 bis 1,0 Gew.-%, oftmals 0,1 bis 0,5 Gew.-%, bezogen auf das Gesamtgewicht der polymeren Formmasse eingesetzt.

In einer Ausführungsform der Erfindung wird im Verfahren in die geschmolzene, polymere Formmasse ein Treibmittel (T) aus der Gruppe aus Pentan, Cyclopentan, Kohlenstoffdioxid und Ethanol oder einer Mischung (zweier oder mehrerer Komponenten) eingebracht.

In einer Ausführungsform der Erfindung wird als Styrol-Polymer-Komponente (S) ein Polystyrol (PS) oder ein Copolymer enthaltend Styrol und Acrylnitril, insbesondere Styrol-Acrylnitril (SAN), eingesetzt. Auch eine Mischung aus PS und anderen Styrol-Polymer-Komponenten ist verwendbar.

Die Erfindung betrifft auch einen Schaumstoff, erhältlich (oder erhalten) durch ein Verfahren nach mindestens einer der o.g. Ausführungsformen.

Dieser Schaumstoff hat vorzugsweise eine Dichte im Bereich von mindestens 30 kg/$m^3$, insbesondere 45-85 kg/$m^3$, oftmals 60-85 kg/$m^3$ (insbesondere bei Polystyrol).

Dieser Schaumstoff hat eine zumindest 50 % geschlossen-zellige Struktur, vorzugsweise eine zu mindestens 80 %, insbesondere zu mindestens 90 %, oftmals zu mindestens 95 % geschlossen-zellige Struktur.

Dieser Schaumstoff hat einen mittlerer Zellendurchmesser (D) von 0,1- 25,0, häufig von 1,0- 16,0, vorzugsweise 2,0-15,0, oftmals 3,0 bis kleiner als 15,0 Mikrometer.

Der Schaumstoff hat vorzugsweise auch eine nahezu einheitliche mittlere Zellengröße (kleine Standardabweichung des Durchmessers) und eine einheitliche Zellenmorphologie, z.B. Waben-förmig, was durch REM-Aufnahmen untersucht werden kann.

Der Schaumstoff ist vorzugsweise ein Polystyrol-basierter Schaumstoff oder ein Styrol-Acrylnitril-Copolymer-basierter Schaumstoff.

Gegenstand der Erfindung ist auch eine Polymerzusammensetzung für die Herstellung eines Schaumstoffs enthaltend mindestens eine Styrol-Polymer-Komponente (S) und mindestens ein Carbonsäureamid der allgemeinen Formel (I) sowie ggf. weitere Additive,

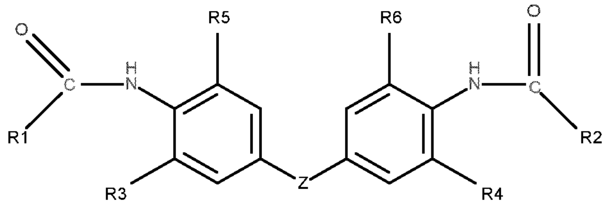

(I)

wobei die Substituenten Z, R1 und R2 sowie R3, R4, R5 und R6 die jeweils oben genannten Definitionen haben.

Die Erfindung betrifft auch die Verwendung eines Carbonsäureamids der allgemeinen Formel (I), wobei die Substituenten Z, R1 und R2 sowie R3, R4, R5 und R6 die jeweils oben genannten Definitionen haben,

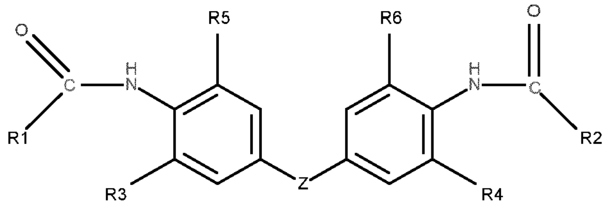

(I)

als ein den mittleren Zellendurchmesser (D) eines Schaums verkleinerndes Additiv bei der Herstellung von Schaumstoffen aus mindestens einem polymeren Material, insbesondere mindestens einer Styrol-Polymer-Komponente (S).

Besonders gute Ergebnisse bzgl. klein-zelliger Schäume, hohem Anteil an geschlossenen Zellen sowie einheitlicher Größe der Zellen werden beim Einsatz von Polystyrol und SAN-Copolymeren erzielt.

Gegenstand der Erfindung ist auch ein Verfahren zur Herstellung eines Carbonsäureamids der allgemeinen Formel (I), wobei die Substituenten Z, R1 und R2 sowie R3, R4, R5 und R6 die jeweils oben genannten Definitionen haben, durch Umsetzung mindestens eines aktivierten Carbonsäure-Derivats mit einem (aromatischen) Bisamin-Derivat.

Gegenstand der Erfindung sind ferner auch die Carbonsäureamide der allgemeinen Formel (I) selbst, insbesondere, wenn Z eine $-CH_2$ Gruppe, R1 und R2 unabhängig Benzyl, Cyclohexyl, n-Butyl, t-Butyl; und R3, R4, R5 und R6 jeweils unabhängig Methyl oder Ethyl bedeuten. Besonders interessant sind die Verbindungen aus den Beispielen.

Mit Hilfe der erfindungsgemäß einsetzbaren Carbonsäureamide der allgemeinen Formel (I) ist es möglich, die Zellgröße eines in dessen Gegenwart hergestellten Schaumstoffes auf einen optimalen Wert einzustellen.

Auch werden Schäume erhalten, die weitgehend (mindestens 80 %, häufig mindestens 90 %, oftmals mindestens 95 %) geschlossene Zellen aufweisen, was sich z.B. durch mikroskopische Untersuchungen nachweisen lässt.

Durch den erfindungsgemäßen Einsatz der Bisamide der Formel (I) wird es möglich, eine deutliche Verkleinerung der Zellen beim Aufschäumen und eine gewünscht einheitliche Morphologie zu erreichen, so dass es möglich ist, verbesserte Formteile aus dem Schaum herzustellen.

Bei dem erfindungsgemäßen Verfahren wird ein Schaumstoff in wenigen Schritten aus wenigstens einem Styrol-haltigen polymeren Material hergestellt. Die eingesetzte Styrol-Polymer-Komponente (S) ist im Allgemeinen ein Homo- oder Copolymer. Geeignete Polymere umfassen Homopolymere aus Styrol-Monomeren (z.B. Styrol, alpha-MethylStyrol, Kern-substituierte Styrole) aber auch Copolymere aus Styrol-Monomeren und damit copolymerisierbaren, ethylenisch ungesättigten Monomeren.

Diese sind z.B. Acrylnitril, Maleinsäureanhydrid, Acrylsäuremethylester, Acrylsäureethylester, Methacrylsäuremethylester, Vinylacetat, Butadien, Divinylbenzol und Butandioldiacrylat. Zum Teil werden diese Co-Monomere nur in geringen Mengen eingesetzt, um ein Schäumen zu gewährleisten.

In einer bevorzugten Ausführungsform ist die Styrol-Polymer-Komponente (S) aus wenigstens 50 Gew.-%, bevorzugt wenigstens 70 Gew.-% Styrol-Monomeren aufgebaut und aus höchstens 50 Gew.-%, bevorzugt höchstens 30 Gew.-% eines weiteren Monomers, z.B. Acrylnitril.

In einer bevorzugten Ausführungsform ist die Styrol-Polymer-Komponente (S) ein Polystyrol. Bevorzugte enthält sie mehr als 95 Gew.-% Polystyrol (PS). Auch Styrol-Acrylnitril (SAN) ist eine bevorzugte Komponente (S), ggf. gemischt mit Polystyrol.

In einer weiteren Ausführungsform wird neben der Styrol-Polymer-Komponente (S) auch bis zu 50 Gew.-%, bezogen auf die gesamte Polymermasse, eines weiteren Polymers verwendet, z.B. eines PMMA oder PVC.

Die erfindungsgemäß einsetzbaren Homo- und Copolymere von Styrol können nach bekannten Verfahren hergestellt werden, beispielsweise durch radikalische oder ionische Polymerisation, in Substanz, Lösung oder Emulsion. Bevorzugt ist die radikalische Polymerisation. Sie haben im Allgemeinen gewichtsgemittelte Molekulargewichte (Mw) von 100.000 bis 400.000 g/Mol, bevorzugt 120.000 bis 350.000 g/Mol.

In Schritt (a) des erfindungsgemäßen Verfahrens wird das polymere Material (S) erwärmt bzw. erhitzt, um eine geschmolzene polymere Formmasse zu erhalten. Dazu ist es notwendig, die Styrol-Polymer-Komponente (S) auf eine Temperatur oberhalb der Schmelz- bzw. Glasübergangstemperatur zu erhitzen. Geeignete Temperaturen betragen im Allgemeinen 100 bis 280 °C, bevorzugt 180 bis 260 °C. Wird Polystyrol eingesetzt, so muss die Komponente (S) i.d.R. auf eine Temperatur 180 °C oder mehr erhitzt werden, um eine Schmelze zu erhalten.

Das Erzeugen einer schäumbaren Polymerschmelze kann in dem Fachmann bekannten Extrudern durchgeführt werden, z.B. über einen Tandem-Aufbau aus Aufschmelzextruder und Sekundärextruder. Der Schritt (a) des erfindungsgemäßen Verfahrens kann kontinuierlich und diskontinuierlich durchgeführt werden, bevorzugt kontinuierlich, bei dem die Styrol-Polymer-Komponente (S) im Aufschmelzextruder aufgeschmolzen wird.

Durch Zugabe eines Treibmittels (T) z.B. im Primär- und/oder Sekundärextruder wird dann eine schäumbare Zusammensetzung gebildet, welche an der Düse einen kontinuierlichen Schaum erzeugen kann.

Der Schritt (b) des erfindungsgemäßen Verfahrens umfasst das Einbringen mindestens eines Treibmittels (T) in die in Schritt (a) geschmolzene Styrol-Polymer-Komponente (S) zur Ausbildung einer schäumbaren Zusammensetzung. Geeignete Treibmittel (T) umfassen insbesondere anorganische und organische Treibmittel. Geeignete anorganische Treibmittel umfassen Kohlenstoffdioxid, Stickstoff, Argon, Wasser, Luft und Helium. Ein häufig eingesetztes Treibmittel ist z.B. eine Mischung von Kohlenstoffdioxid und Wasser.

Organische Treibmittel sind z.B. aliphatische Kohlenwasserstoffe mit 1-9 Kohlenstoffatomen und vollkommen oder partiell halogenierte aliphatische Kohlenwasserstoffe mit 1-4 Kohlenstoffatomen. Aliphatische Kohlenwasserstoffe sind z.B. Methan, Ethan, Propan, n-Butan, iso-Buten, n-Pentan, iso-Pentan, und Neopentan. Vollständig und partiell halogenierte aliphatische Kohlenwasserstoffe sind insbesondere Fluorkohlenstoffverbindungen, Chlorkohlenstoffverbindungen und Chlorfluorkohlenstoffverbindungen. Beispiele für Fluorkohlenstoffverbindungen sind Methylfluorid, Perfluoromethan, Ethylfluorid, Difluormethan, 1,1-Difluorethan, 1,1,1-Trifluorethan, 1,1,1,2-Tetrafluorethan, Pentafluorethan, Difluormethan, Perfluorethan, 2,2-Difluorpropan, 1,1,1-Trifluorpropan, Perfluorpropan, Difluorpropan, Difluorpropan, Perfluorbutan, Perfluorcyclopentan.

Teilweise halogenierte Chlorkohlenstoffverbindungen und Chlorfluorkohlenstoffverbindungen, welche zum Einsatz in dem erfindungsgemäßen Verfahren geeignet sind, umfassen Methylchlorid, Methylenchlorid, Ethylchlorid, 1,1,1-Trichlorethan, Chlordifluormethan, 1,1-Dichlor-1-fluorethan, 1-Chlor-1,1-difluorethan, 1,1-Dichlor-2,2,2-trifluorethan und 1-Chlor-1,2,2,2-tetrafluorethan. Vollständig halogenierte Chlorfluorkohlenwasserstoffverbindungen umfassen Trichlormonofluormethan, Dichlordifluormethan, Trichlortrifluorethan, 1,1,1-Trifluorethan, Pentafluorethan, Dichlor-tetrafluorethan, Chlorheptafluorpropan und Dichlorhexafluorpropan.

Weitere geeignete chemische Treibmittel (T) sind Azodicarbonsäurediamid, Azodiisobutyronitril, Benzolsulfonhydrazid, 4,4-Oxibenzolsulfonylsemicarbazid, p-Toluolsulfonylsemicarbazid, Bariumazodicarboxylat, N,N'-Dimethyl-N,N'-Dinitroso-therephthalamid und Trihydrazinotriazin.

Ein weiteres Treibmittelgemisch umfasst 20 bis 95 Gew.-% Kohlenstoffdioxid, 5 bis 80 Gew.-% Wasser und 0 bis 75 Gew.-% eines Alkohols, beispielsweise Methanol oder Ethanol, eines Ketons oder Ethers.

Aus Umweltschutzgründen ist es oftmals wünschenswert anorganische Treibmittel zu verwenden. Zwei beliebte anorganische Treibmittel sind Kohlenstoffdioxid und Wasser.

Die Menge des Treibmittels (T), welche in die geschmolzene Styrol-Polymer-Komponente (S) eingebracht wird, um eine schäumbare Zusammensetzung (Z) zu erhalten, beträgt 0,1-20, bevorzugt 0,5-15, oftmals 1,0 -10 Gew.-% bezogen auf die Masse der Styrol-Polymer-Komponente (S).

Das Treibmittel kann in die Styrol-Polymer-Komponente (S) durch verschiedene bekannte Methoden eingebracht werden, beispielsweise mittels eines Extruders, eines Mixers oder eines Blenders. Das Treibmittel wird z.B. mit dem geschmolzenen Polymermaterial bei erhöhtem Druck gemischt. Der Druck ist hoch genug, um im Wesentlichen eine Ausdehnung des geschmolzenen Polymermaterials zu vermeiden und um eine homogene Verteilung des Treibmittels (T) in der geschmolzenen Styrol-Polymer-Komponente (S) zu ermöglichen. Geeignete Drücke sind z.B. 100 bis 200 bar (absolut), bevorzugt 150 bis 170 bar (absolut).

Die Temperatur in Schritt (b) des erfindungsgemäßen Verfahrens ist so gewählt, dass das polymere Material im geschmolzenen Zustand vorliegt, Daher wird Schritt (b) des erfindungsgemäßen Verfahrens im Allgemeinen bei 100-180 °C durchgeführt. Schritt (b) kann kontinuierlich oder diskontinuierlich durchgeführt werden, bevorzugt kontinuierlich.

In einer bevorzugten Ausführungsform wird der geschmolzenen Styrol-Polymer-Komponente (S) wenigstens ein Carbonsäureamid-Derivat wie oben beschrieben zugesetzt.

Die Zugabe der Bisamide und weiterer Additive kann z.B. in Schritt (a) und/oder (b) des Verfahrens erfolgen. Sie kann als Pulver-Pulver-Mischung oder z.B. über ein MasterBatch erfolgen, sowohl in Schritt (a) als auch in Schritt (b), bevorzugt in Schritt (a).

Der Schritt (c) des erfindungsgemäßen Verfahrens umfasst das Aufschäumen der schäumbaren Zusammensetzung (Z), um einen geschäumten Formkörper (den Schaumstoff) zu erhalten.

In einer bevorzugten Ausführungsform wird dieses Aufschäumen durch Extrusion der Treibmittel (T)- haltigen, geschmolzenen Styrol-Polymer-Komponente (S) durch eine geeignete Vorrichtung, beispielsweise eine Düse, in einen Bereich niedrigeren Druckes (als in Schritt (b) eingesetzt) erhalten. Schritt (c) wird ebenfalls bei einer Temperatur durchgeführt, bei der die zu schäumende Zusammensetzung in geschmolzenem Zustand vorliegt, im Allgemeinen 80-125 °C, bevorzugt 110-125 °C.

Dadurch, dass das ein Treibmittel enthaltende, geschmolzene, polymere Material in Schritt (c) in einen Bereich überführt wird, in dem ein niedrigerer Druck herrscht, wird das Treibmittel in den gasförmigen Zustand überführt. Durch den großen Volumenanstieg wird das polymere Material ausgedehnt und aufgeschäumt.

Das erfindungsgemäße Verfahren ist dadurch gekennzeichnet, dass in der geschmolzenen die Styrol-Polymer-Komponente (S) ein Carbonsäurebisamid der oben genannten allgemeinen Formel (I) eingesetzt wird.

In einer besonders bevorzugten Ausführungsform ist das Carbonsäurebisamid ausgewählt aus der Gruppe der in den Beispielen genannten Verbindungen und Mischungen davon.

Die erfindungsgemäß einsetzbaren Carbonsäurebisamide der Formel (I) können nach dem Fachmann bekannten Verfahren erhalten werden, beispielsweise durch, gegebenenfalls katalysierte, Umsetzung der entsprechenden Carbonsäuren (bzw. aktivierten Derivate) mit den entsprechenden Aminen.

Das wenigstens ein Carbonsäurebisamid (I) wird im erfindungsgemäßen Verfahren im Allgemeinen in einer Menge von 0,01 bis 2 Gew.-% eingesetzt, bevorzugt 0,1 bis 2 Gew.-%, besonders bevorzugt 0,2 bis 1,0 Gew.-%, beispielsweise 0,1 bis 0,5 Gew.-%, jeweils bezogen auf das Gesamtgewicht der polymeren Formmasse.

Das wenigstens eine Carbonsäurebisamid der Formel (I) kann der polymeren Formmasse in den Schritten (a) oder (b) des erfindungsgemäßen Verfahrens zugesetzt werden. Geeignete Vorgehensweisen sind z.B. die Dosierung des Carbonsäurebisamids in die Aufschmelzzone des Extruders zum polymeren Material.

Der durch das erfindungsgemäße Verfahren erhältliche Schaumstoff zeichnet sich durch eine reduzierte Größe der Zellen aus.

Im Allgemeinen liegen in dem erfindungsgemäß hergestellten Schaum sehr viele Zellen (>150) pro mm (jeder Raumrichtung) des Schaumstoffs vor.

Die kleine mittlere Zellengröße des Schaums kann durch dem Fachmann bekannte Verfahren gemessen werden, beispielsweise Rasterelektronenmikroskopie. Die erwähnte kleine Zellgröße und die homogene Größenverteilung ist auf den Einsatz der speziellen Carbonsäureamide der Formel (I) im erfindungsgemäßen Verfahren zurückzuführen.

Durch die eingesetzten Carbonsäureamide kann das polymere Material besonders gut aufgeschäumt werden, so dass z.B. auch Schaumstoffplatten mit größeren Dicken (z.B. größer 60 mm) direkt erhältlich sind. Diese haben gute Dämmeigenschaften.

Im erfindungsgemäßen Verfahren ist es möglich, ggfs. weitere Additive in den Schaumstoff einzubringen, beispielsweise anorganische Füllmittel, Pigmente, Antioxidantien, Säurefänger, UV-Absorber, Flammschutzmittel, Bearbeitungshilfsmittel und Extrusionshilfsmittel. Diese weiteren Additive werden im Allgemeinen in einer Menge von 0,1 bis 2 Gew.-% eingesetzt, jeweils bezogen auf die gesamte polymere Formmasse. Diese Additive können bereits vor Schritt (a) im polymeren Material vorliegen, oder sie werden in Schritt (a) und/oder (b) dem polymeren Material zugegeben.

Als (weitere) Nukleierungsmittel können feinteilige, anorganische Feststoffe wie Talkum, Metalloxide, Silikate oder Polyethylenwachse in Mengen von im Allgemeinen 0,1 bis 1,0 Gew-%, bezogen auf die Styrol-Polymer-Komponente (S) eingesetzt werden. Der mittlere Teilchendurchmesser dieses Nukleierungsmittels liegt in der Regel im Bereich von 0,01 bis 100 $\mu$m, bevorzugt 20 bis 60 $\mu$m. Das Nukleierungsmittel kann nach bekannten Methoden der Polymerschmelze zugegeben werden.

Der nach dem vorliegenden Verfahren hergestellte Schaumstoff kann auch dazu verwendet werden, Oberflächen zu isolieren, indem eine isolierende Schicht aus dem erfindungsgemäß hergestellten Schaumstoff auf diese Oberfläche aufgebracht wird. Dies kann in allen bekannten Isolierungsanwendungen durchgeführt werden, beispielsweise an Dächern, Gebäuden und Haushaltsgeräten, wie Kühlschränken.

Der erfindungsgemäß hergestellte Schaumstoff kann in eine Vielzahl von geschäumten Formteilen geformt werden, beispielsweise für Verpackungen, oder in massive Werkstücke geeigneter Größe.

Die vorliegende Erfindung betrifft auch die Verwendung von wenigstens einem Carbonsäure-bisamid der allgemeinen Formel (I) mit den oben definierten Bedeutungen beim Aufschäumen von wenigstens einer Styrol-Polymer-Komponente (S).

Die vorliegende Erfindung betrifft auch einen Schaumstoff, der durch das erfindungsgemäße Verfahren erhältlich (bzw. erhalten) ist. Bei den Untersuchungen der Schäume hinsichtlich ihrer Morphologie, Größe und physikalischen Eigenschaften wurden viele Messergebnisse gewonnen. Exemplarisch werden die nachfolgenden Figuren erläutert.

**Figur 1** zeigt zwei rasterelektronenmikroskopische Aufnahmen (A bei 50-facher Vergrößerung und B bei 250-facher Vergrößerung) von Zellen eines (reinen) Polystyrol-Schaumstoffs ("Neat PS"), der ohne Zusatz eines Bisamids der Formel (I) hergestellt wurde. Der Schaum basiert auf handelsüblichem Polystyrol PS 168N (INEOS STRYROLUTION, Frankfurt). Es ist deutlich zu erkennen, dass die Zellen des Schaums eine große mittlere Zellengröße aufweisen. Die Zellgrößenverteilung des PS-Schaums ist zudem uneinheitlich.

**Figur 2** zeigt zwei rasterelektronenmikroskopische Aufnahmen (A bei 50-facher Vergrößerung und B bei 250-facher Vergrößerung) von Zellen eines Polystyrol-Schaumstoffs, der unter Einsatz des Bisamids der Formel (I) hergestellt wurde. Der Schaum basiert auf Polystyrol PS168N (INEOS STRYROLUTION). Dabei wurde als Komponente (3b) die Verbindung N,N'-[Methylenbis(2,6-diethyl-4,1-phenylen)]bis-cyclohexan-carboxamid] als Additiv in einer Menge von 0,1 Gewichtsprozent eingesetzt. Man sieht deutlich, dass die Morphologie der Zellen des Schaums geschlossen-zellig und kleinzellig ist. Man erkennt eine kleine mittlere Zellengröße (14,4 +/- 4,7 Mikrometer) und eine enge Zellgrößenverteilung.

Der Polystyrol-Schaum hat dabei eine Zell-Dichte von $4{,}3 \times 10^8$ $cm^{-3}$ sowie eine Schaumdichte von 76,5 $kg/m^3$. Man findet auch keine ausgeprägte bimodale Zellgrö-ßenverteilung im Schaum, wie es oftmals bei anderen Additiven, wie z.B. Trisamid-Derivaten auftritt.

Die Erfindung wird ferner durch die nachfolgenden Beispiele und Ansprüche näher erläutert.

**Beispiele**

Die verwendeten "gewinkelten" Carbonsäurebisamid-Derivate können gemäß den unten beschriebenen Verfahren synthetisiert werden, alle Chemikalien für die Herstellung sind kommerziell erhältlich und können ohne weitere Aufreinigung eingesetzt werden. Das Silikonöl M100 mittlerer Viskosität (Carl Roth GmbH + Co. KG) wurde als Ölbad für das Aufschäumen der Proben im Batchschaum-Verfahren verwendet. Das Treibmittel $CO_2$ mit 99,995 % Reinheit wurde von Rießner Gase GmbH bezogen.

Zur Charakterisierung wurden folgende analytische Verfahren und Methoden eingesetzt:
Differential-Scanning-Kalorimetrie (DSC): DSC-Untersuchungen wurden mit einem Mettler Toledo DSC 2 durchgeführt. Hierzu wurden ca. 6 - 12 mg einer Verbindung in einem 30 $\mu$L Hochdruck-Tiegel eingewogen. Die gewinkelten Bisamid-Derivate 1a-d, 2a, 2d, 3a und 3d wurden in einem Temperaturbereich von 25-300 °C und die gewinkelten Bisamid-Derivate 2b-c und 3b-c in einem Temperaturbereich von 25-350 °C jeweils mit einer Rate von 10 K/min vermessen. Jeder Heiz- und Kühlschritt wurde dreimal wiederholt. Die dokumentierten Schmelzpunkte wurden dem zweiten Heizschritt entnommen.

Massenspektrometrie (MS): MS-Untersuchungen wurden mittels Elektronensprayionisation an einem üblichen Gerät (FINNIGAN MAT 8500 Spektrometer von Thermo-Fisher Scientific) durchgeführt.

Rasterelektronenmikroskopie (REM): Rasterelektronenmikroskopische Aufnahmen wurden an einem üblichen Mikroskop (Zeiss LEO 1530) mit einer Beschleunigungsspannung von 3 kV und unter Verwendung eines Innenlinsen- oder SE2-Detektors gemacht.

Hierzu wurden zunächst die Schaumproben mit flüssigem Stickstoff kryo-frakturiert und die Bruchkanten mit 2 nm Platin unter Argon-Atmosphäre mit einem Coater (Cressington Sputer Coater 208HR) besputtert. Vor dem Sputtern wurden die Proben an den Seiten zusätzlich mit selbstklebender Kupferfolie ummantelt, um eine bessere Leitfähigkeit zu gewährleisten.

Thermische Leitfähigkeit: Die thermische Leitfähigkeit der Schaumproben wurde mit einem üblichen Wärmeflussmesser (LaserComp FOX 50 von TA Instruments) gemessen. Die Schaumproben wurden in Zylinder mit 60 mm Durchmesser und einer Dicke (L) zwischen 3 mm und 8 mm, abhängig von der extrudierten Dicke des Schaumstoffs, geschnitten. Die Proben wurden zwischen zwei temperierten Platten positioniert. Dabei wurde die Temperatur der oberen Platte auf 30 °C und die der unteren Platte auf 20 °C eingestellt, so dass entlang der Probendicke eine Temperaturdifferenz ($\Delta$T) von 10 °C entsteht. Der resultierende Wärmefluss (Q/A) durch die Schaumprobe wurde durch zwei Dünnschicht-Wärmeflusswandler gemessen. Die thermischen Leitfähigkeiten ($\lambda_t$) wurden gemäß Formel (1) berechnet:

$$\lambda_t = \frac{Q \cdot L}{A \cdot \Delta T} \qquad (1)$$

Mindestens fünf Proben jedes Schaums wurden in verschiedenen Positionen gemessen und Durchschnittswerte der thermischen Leitfähigkeit bestimmt.

Schaumdichte: Die Schaumdichte wurde durch das Wasserverdrängungsverfahren nach der Norm ISO 1183 unter Verwendung einer Analysenwaage (Mettler Toledo XP 205) mit Dichte-Kit bestimmt. Dafür wurden kleine Quader aus den Proben geschnitten und an Luft gewogen ($m_{Luft}$). Danach wurde der Auftrieb der Proben unter Wasser bestimmt.

($m_{Wasser}$; $\rho_{Wasser}$: Dichte des Wassers bei Messtemperatur). Die resultierende Dichte ($\rho_{Schaum}$) wurde mit der folgenden Gleichung berechnet:

$$\rho_{Schaum} = \frac{m_{Luft}}{m_{Luft} - m_{Wasser}} \cdot \rho_{Wasser}$$

Jede Messung wurde mit drei verschiedenen Quadern der entsprechenden Probe durchgeführt und der Durchschnittswert dokumentiert.

Morphologie: Die Morphologie der Schaumproben wurde mittels REM-Aufnahme untersucht. Ein Bereich ($A_{Zelle}$) von mindestens 70 Zellen jeder Probe wurde berücksichtigt. Unter Annahme einer Kreisform der Zellen wurde die folgende Gleichung zur Bestimmung der Größe ($\phi$) aller einzelnen Zellen herangezogen:

$$\phi = 2 \cdot \sqrt{\frac{A_{Zelle}}{\pi}}$$

Das arithmetische Mittel ($\phi_m$) aller berechneten Zellen mit Standardabweichung ist für jeden Schaum aufgeführt.

**Beispiel 1 Herstellung der Carbonsäurebisamid-Derivate der Formel (I)**

Ausgehend von den entsprechenden aromatischen Bisamin-Verbindungen und Säurederivaten wurden die folgenden aromatischen Bisamid-Derivate hergestellt, die bei Verarbeitungstemperatur im Polymer (PS, SAN) gut löslich sind.

Die Verbindungen 1a-d, 2a-d, 3a-d wurden gereinigt und charakterisiert. Die Synthesen dieser Bisamid-Additive der Formel (I) werden nachfolgend beschrieben.

**1a Synthese von N,N'-[Methylenbis(4,1-phenylen)]bis[benzamid]:**

5 g (25,2 mmol) 4,4'-Diaminodiphenylmethan, 4,5 mL Pyridin und 100 mL NMP wurden in einem Schlenkkolben gemischt und auf ca. 0-5°C abgekühlt. Unter Argon-Atmosphäre wurden 7,79 g (55,4 mmol) Benzoylchlorid tropfenweise zugefügt und die Mischung anschließend auf Raumtemperatur erwärmt. Nach einer Stunde wurde das Reaktionsgemisch in Eiswasser gefällt und der erhaltene Feststoff abfiltriert und getrocknet. Zur weiteren Aufreinigung wurde der Feststoff in 500 mL MeOH unter Rückfluss erhitzt, filtriert und im Hochvakuum getrocknet. 9,65 g (95 %) des Produkts 1a wurden in Form eines weißen Pulvers erhalten. Charakterisierung: MS:(70 eV), m/z (%): 406 [M+]; DSC: $T_m$ = 249 °C.

**1b Synthese von N,N'-[Methylenbis(4,1-phenylen)]bis[cyclohexancarboxamid]:**

3 g (15,0 mmol) 4,4'-Diaminodiphenylmethane, 20 mL Pyridin, 100 mL NMP und LiCl wurden in einem Schlenkkolben gemischt und auf ca. 0-5°C abgekühlt. Unter Argon-Atmosphäre wurden 4,9 g (33 mmol) Cyclohexancarbonylchlorid tropfenweise zugefügt. Das Reaktionsgemisch wurde anschließend für 12h auf 80 °C erhitzt und anschließend in Eiswasser ausgefällt. Der erhaltene Feststoff wurde abfiltriert und getrocknet. Zur weiteren Aufreinigung wurde der Feststoff in 500 mL MeOH umkristallisiert, filtriert und im Hochvakuum getrocknet. 5,5 g (88 %) des Produkts 1b wurden in Form eines weißen Pulvers erhalten. Charakterisierung: (88 %); MS: (70 eV), m/z (%): 418 [M+]; DSC: $T_m$ = 221 °C.

**1c Synthese von N,N'-[Methylenbis(4,1-phenylen)]bis[2,2-dimethylpropanamid]:**

3 g (15 mmol) 4,4'-Diaminodiphenylmethan, 20 mL Pyridine, 100 mL NMP und LiCl wurden in einem Schlenkkolben gemischt und auf ca. 0-5°C abgekühlt. Unter Argon-Atmosphäre wurden 4 g (33 mmol) Pivaloylchlorid tropfenweise zugefügt. Die Reaktion wurde für 12 h bei 80 °C gerührt und anschließend in Eiswasser gefällt. Der erhaltene Feststoff wurde abfiltriert und getrocknet. Zur weiteren Aufreinigung wurde der Feststoff in 500 mL Ethylacetat umkristallisiert, abfiltriert und im Hochvakuum getrocknet. 4,1 g (75 %) des Produkts 1c wurden in Form eines weißen Pulvers erhalten. Charakterisierung: MS: (70 eV), m/z (%): 366 [M+]; DSC: $T_m$ = 239 °C

**1d Synthese von N,N'-[Methylenbis(4,1-phenylen)]bis[pentanamid]:**

5 g (25,21 mmol) 4,4'-Diaminodiphenylmethan, 4,5 mL Pyridine und 100 mL NMP wurden in einem Schlenkkolben gemischt und auf ca. 0-5°C abgekühlt. Unter Argon-Atmosphäre wurden 6,02 g (55,47 mmol) Valeroylchlorid tropfenweise zugefügt und die Mischung auf Raumtemperatur erwärmt. Nach einer Stunde Reaktionszeit wurde das Gemisch in Eiswasser gefällt. Der erhaltene Feststoff wurde daraufhin abfiltriert und getrocknet. Zur weiteren Aufreinigung wurde der Feststoff in 300 mL MeOH umkristallisiert, filtriert und im Hochvakuum getrocknet. 8,8 g (95 %) des Produkts 1d wurden in Form eines weißen Pulvers erhalten. Charakterisierung: MS: (70 eV), m/z (%): 366 [M+]; DSC: $T_m$ = 192 °C

**2a Synthese von N,N'-[Methylenbis(2,6-dimethyl-4,1-phenylen)]bis[benzamid]:**

5 g (19,65 mmol) 4,4'Methylenbis(2,6-dimethylanilin), 3,5 mL Pyridin und 100 mL NMP wurden in einem Schlenkkolben gemischt und auf ca. 0-5°C abgekühlt. Unter Argon-Atmosphäre wurden 6,07 g (43,23 mmol) Benzoylchlorid tropfenweise zugefügt und die Mischung auf Raumtemperatur erwärmt. Nach einer Stunde wurde das Reaktionsgemisch in Eiswasser gefällt. Der erhaltene Feststoff wurde daraufhin abfiltriert und getrocknet. Zur weiteren Aufreinigung wurde der Feststoff in 500 mL MeOH unter Rückfluss erhitzt, filtriert und im Hochvakuum getrocknet. 7,5 g (82 %) des Produkts 2a wurden in Form eines weißen Pulvers erhalten. Charakterisierung: MS: (70 eV), m/z (%): 462 [M+]; DSC: $T_m$ = 225 °C

**2b Synthese von N,N'-[Methylenbis(2,6-dimethyl-4,1-phenylen)]bis[cyclohexan-carboxamid]:**

3,56 g (14,00 mmol) 4,4'Methylenbis(2,6-dimethylanilin), 4,28 mL $Et_3N$ und 100 mL THF wurden in einem Schlenkkolben gemischt und auf ca. 0-5°C abgekühlt. Unter Argon-Atmosphäre wurden 4,51 g (30,76 mmol) Cyclohexancarbonylchlorid tropfenweise zugefügt. Nach 48 h bei 60 °C wurde das Reaktionsgemisch in Eiswasser gefällt. Der erhaltene Feststoff wurde daraufhin abfiltriert mit Wasser gewaschen und anschließend getrocknet. Zur weiteren Aufreinigung wurde der Feststoff in 250 mL DMF umkristallisiert, filtriert und im Hochvakuum getrocknet.

5,6 g (84 %) des Produkts 2b (Formel (II)) wurden in Form eines weißen Pulvers erhalten. Charakterisierung: MS: (70 eV), m/z (%): 474 [M+]; DSC: $T_m$ = 307 °C.

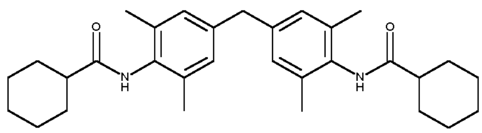

(II)

**2c Synthese von N,N'-[Methylenbis(2,6-dimethyl-4,1-phenylen)]bis[2,2-dimethyl-propanamid]:**

4 g (15,72 mmol) 4,4'Methylenbis(2,6-dimethylanilin), 2,8 mL Pyridin und 100 mL THF wurden in einem Schlenkkolben gemischt und auf ca. 0-5°C abgekühlt. Unter Argon-Atmosphäre wurden 4,17 g (34,59 mmol) Pivaloylchlorid tropfenweise zugefügt und die Mischung auf Raumtemperatur erwärmt. Nach einer Stunde wurde das Reaktionsgemisch in Eiswasser gefällt. Der Feststoff wurde daraufhin abfiltriert und getrocknet. Zur weiteren Aufreinigung wurde der Feststoff zuerst in 100 mL MeOH umkristallisiert, gefolgt von einer Filtration über Kieselgel mit DMF als Laufmittel. Das Lösungsmittel wurde eingeengt, der Feststoff in Wasser ausgefällt und bei 80 °C getrocknet. 4,9 g (74 %) des Produkts 2c wurden in Form eines weißen Pulvers erhalten. Charakterisierung: MS: (70 eV), m/z (%): 422 [M+]; DSC: $T_m$ = 310 °C

**2d Synthese von N,N'-[Methylenbis(2,6-dimethyl-4,1-phenylen)]bis[pentanamid]:**

3 g (11,79 mmol) 4,4'Methylenbis(2,6-dimethylanilin), 2,4 mL Pyridine und 200 mL THF wurden in einem Schlenkkolben gemischt und auf ca. 0-5°C abgekühlt. Unter Argon-Atmosphäre wurden 2,81 g (25,94 mmol) Valeroylchlorid tropfenweise zugefügt und die Mischung auf Raumtemperatur erwärmt. Nach einer Stunde wurde das Reaktionsgemisch in Eiswasser gefällt. Der Feststoff wurde daraufhin abfiltriert und getrocknet. Zur Aufreinigung wurde der Feststoff in 300 mL MeOH umkristallisiert, filtriert und im Hochvakuum getrocknet. 3,6 g (72 %) des Produkts 2d wurden in Form eines weißen Pulvers erhalten. Charakterisierung: MS: (70 eV), m/z (%): 422 [$M^+$]; DSC: $T_{m1}$ = 190 °C, $T_{m2}$ = 258 °C

**3a Synthese von N,N'-[Methylenbis(2,6-diethyl-4,1-phenylen)]bis[benzamid]:**

5 g (16,10 mmol) 4,4'Methylenbis(2,6-diethylanilin), 2,8 mL Pyridine und 100 mL NMP wurden in einem Schlenkkolben gemischt und auf ca. 0-5°C abgekühlt.

Unter Argon-Atmosphäre wurden 4,98 g (35,42 mmol) Benzoylchlorid tropfenweise zugefügt und die Mischung auf Raumtemperatur erwärmt. Nach einer Stunde wurde das Reaktionsgemisch in Eiswasser gefällt. Der erhaltene Feststoff wurde anschließend abfiltriert und getrocknet. Zur weiteren Aufreinigung wurde der Feststoff in 500 mL MeOH unter Rückfluss erhitzt, filtriert und im Hochvakuum getrocknet. 7,0 g (83 %) des Produkts 3a wurden in Form eines weißen Pulvers erhalten. Charakterisierung: MS: (70 eV), m/z (%): 518 [M+]; DSC: $T_m$ = 258 °C

**3b Synthese von N,N'-[Methylenbis(2,6-diethyl-4,1-phenylen)]bis-[cyclohexan-carboxamid]:**

9 g (28,00 mmol) 4,4'Methylenbis(2,6-diethylanilin), 5,1 mL Pyridine und 120 mL NMP wurden in einem Schlenkkolben gemischt und auf ca. 0-5°C abgekühlt. Unter Argon-Atmosphäre und eisgekühlt wurden 9,34 g (63,00 mmol) Cyclohexancarbonylchlorid tropfenweise zugefügt und die Mischung auf Raumtemperatur erwärmt. Nach zwei Stunden Reaktionszeit wurden der Mischung 100 mL Wasser hinzugefügt, für eine weitere Stunde gerührt und anschließend der Feststoff filtriert. Zur weiteren Aufreinigung wurde der Feststoff in 200 mL Aceton unter Rückfluss erhitzt, filtriert und im Hochvakuum getrocknet. 14,2 g (92 %) des Produkts 3b (Formel (III)) wurden in Form eines weißen Pulvers erhalten. Charakterisierung: MS: (70 eV), m/z (%): 530 [$M^+$]; DSC: $T_m$ = 297 °C

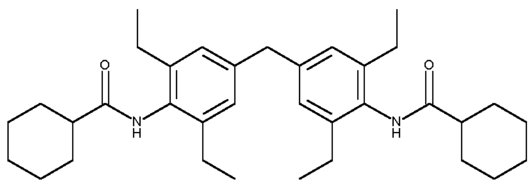

(III)

**3c Synthese von N,N'-[Methylenbis(2,6-diethyl-4,1-phenylen)]bis[2,2-dimethyl-propanamid]:**

4,34 g (14,00 mmol) 4,4'Methylenbis(2,6-diethylanilin), 4,3 mL $Et_3N$ und 50 mL THF wurden in einem Schlenkkolben gemischt und auf ca. 0-5°C abgekühlt. Unter Argon-Atmosphäre wurden 3,71 g (30,80 mmol) Pivaloylchlorid tropfenweise zugefügt und das Reaktionsgemisch auf 60°C erhitzt. Nach 48 h wurde das Gemisch in Eiswasser gefällt. Der erhaltene Feststoff wurde daraufhin abfiltriert und in 500 mL MeOH umkristallisiert. Zur weiteren Aufreinigung wurde der Feststoff in 250 mL DMF umkristallisiert, filtriert und im Hochvakuum getrocknet. 4,8 g (71 %) des Produkts 3c wurden in Form eines weißen Pulvers erhalten. Charakterisierung: MS: (70 eV), m/z (%): 478 [$M^+$]; DSC: $T_m$ = 328 °C

**3d Synthese von N,N'-[Methylenbis(2,6-diethyl-4,1-phenylen)]bis[pentanamid]:**

4,34 g (14,00 mmol) 4,4'Methylenbis(2,6-diethylanilin), 4,3 mL $Et_3N$ und 50 mL THF wurden in einem Schlenkkolben zusammengemischt und auf ca. 0-5°C abgekühlt. Unter Argon-Atmosphäre wurden 3,71 g (30,8 mmol) Valeroylchlorid tropfenweise zugefügt und anschließend auf 50°C erhitzt. Nach 3 h wurde das Reaktionsgemisch in Eiswasser gefällt. Der erhaltene Feststoff wurde daraufhin abfiltriert und mit $H_2O$ gewaschen. Zur weiteren Aufreinigung wurde der Feststoff in 200 mL MeOH umkristallisiert, filtriert und im Hochvakuum getrocknet. 3,7 g (56 %) des Produkts 3d (Formel (IV)) wurden in Form eines weißen Pulvers erhalten. Charakterisierung: MS: (70 eV), m/z (%): 478 [M+]; DSC: $T_{m1}$ = 120 °C, $T_{m2}$ = 220 °C

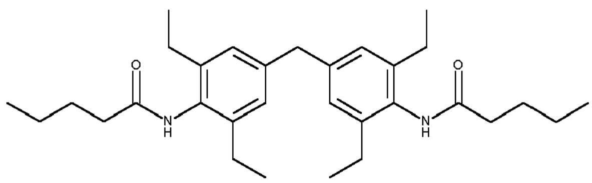

(IV)

Alle in nachfolgender Tabelle 1 abgebildeten Carbonsäure-bisamide unterfallen der Formel (I). Sie lassen sich auch in größeren Mengen gut herstellen. Die genannten Verbindungen sind in Polystyrol und SAN bei Verarbeitungstemperatur löslich.

**Tabelle 1** Verwendete gewinkelte Bisamide der allgemeinen Formel (I)

| Bisamid-Additiv | | Substituenten $R_3$,$R_4$ $R_5$, $R_6$ | Substituenten $R_1$ , $R_2$ |
|---|---|---|---|
| 1 | a | H | Benzyl |
| | b | | Cyclohexyl |
| | c | | t-Butyl |
| | d | | n-Butyl |
| 2 | a | Methyl | Benzyl |
| | b | | Cyclohexyl |
| | c | | t-Butyl |
| | d | | n-Butyl |
| 3 | a | Ethyl | Benzyl |
| | B | | Cyclohexyl |
| | C | | t-Butyl |
| | D | | n-Butyl |

**Beispiel 2 Vergleichs-Carbonsäureamid-Derivaten**

Für die Vergleichsexperimente wurden zum einen das Trisamid-Additiv Irgaclear® XT 386 (BASF SE) und zum anderen das Bisamid-Additiv NJ Star NU100 (N,N'-Dicyclohexyl-2,6-naphthalindicarboaxmid) der New Japan Chemical verwendet.

Das als Transparenz-Verstärker für Polypropylen eingesetzte symmetrische aromatische Trisamid Irgaclear® XT 386 (1,3,5-Tris(2,2-dimethyl-proprionyl-amino)-benzol; BASF, Formel (V)) kann analog aus 1,3,5-Tris-aminobenzol und Säurechlorid hergestellt werden.

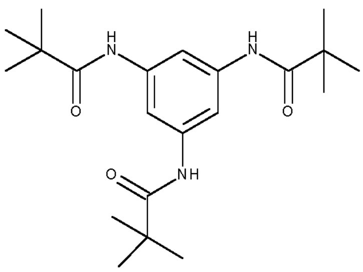

(V)

Ein (nicht-gewinkeltes) aromatisches Bis-amid-Derivat kann aus Naphthalin-2,6-dicarbon-säure und Cyclohexylamin hergestellt werden. Es wird auch von New Japan Chemical als Produkt NJ STAR NU100 (Formel (VII)) vertrieben.

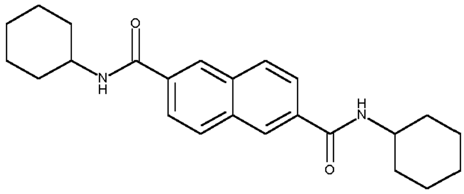

(VII)

**Beispiel 3 Herstellung der Schäume**

Es wurden Polystyrol-Schaumstoffe nach dem erfindungsgemäßen Verfahren hergestellt. Dazu wurde ein handelsübliches Polystyrol (PS168N, INEOS STRYROLUTION, Frankfurt am Main) in Form von 3 mm Zylindergranulat (3 × 2 mm) verwendet. Für das Polystyrol wurde ein mittleres Molekulargewicht Mw von 340.000 g/mol ermittelt.

a) Herstellung der Polymerpulver/Additiv-Pulver-Mischungen (Masterbatch)

Hierzu wurde zunächst das Polymer-Granulat mit einer Ultra-Zentrifuge (mill Retsch ZM200) bei 18.000 rpm Umdrehungsgeschwindigkeit und einer Siebmaschengröße von 1000 μm gemahlen, um die weitere Einarbeitung und die Verteilung des Additivs zu gewährleisten. Während des Mahlvorgangs wurde das Polymer mit flüssigem Stickstoff gekühlt.

Anschließend wurde das gemahlene PS für jeden Pulver-Pulver-Masterbatch mit 1,0 Gew.-% des entsprechenden Additivs versehen und mit einem Heidolph Reax 2-Mixer bei 50 rpm homogenisiert.

b) Batchschaumverfahren:

Compoundierung von Polymer-Additiv-Konzentrationsreihen und Spritzguss zu Probenkörpern

Die Aufbereitung wurde mit einem gleichsinnig drehenden Doppelschnecken-Compounder (DSM Xplore 15 mL) durchgeführt. Die Komponenten wurden für 5 Minuten mit einer Drehgeschwindigkeit von 50 rpm bei 260 °C vermischt. Danach wurde die Polymer-Schmelze 2 Minuten lang in den Spritzgussbehälter abgelassen. Da es aufwändig ist, den Extruder vollständig zu entleeren und ein definiertes Totvolumen zurückbleibt, ist es von Vorteil, eine Verdünnungsreihe anzusetzen, um verschiedene Konzentrationen zu ermöglichen. Anfangs ist der Extruder mit 13,5 g des entsprechenden Materials gefüllt. Etwa 8,1 g können in den Spritzgussbehälter überführt werden, während 5,4 g im Compounder zurückbleiben. Mit diesem Wissen ist es möglich, die gewünschten Konzentrationen zu erzielen.

Die folgenden acht Konzentrationen der o.g. Additive in der Polymerzusammensetzung wurden hergestellt: 1,0; 0,75; 0,5; 0,25; 0,1; 0,05; 0,025 und 0,01 Gew.-%.

Das Spritzgießen wurde mit der Mikro-Spritzgussmaschine DSM Xplore 12 mL durchgeführt. Der Behälter hatte eine Temperatur von 250 °C und die Schmelze wurde mit einem Druck von 6 bar für 10 Sekunden eingespritzt. Der Druck wurde für weitere 10 Sekunden gehalten. Es wurden runde Polymer-Plättchen mit 27 mm Durchmesser und 1,1 mm Dicke erhalten und getestet. Um innere Spannungen der Polymerproben aus dem Spritzguss-Verfahren zu eliminieren, werden diese in einer geschlossenen Eisenform bei 135 °C für 4 Stunden getempert. Die spannungsfreien Proben garantieren ein einheitliches Aufschäumen.

Sättigung und Aufschäumen der Polymer-Probenkörper im Batch-Schaumverfahren

Nach dem Tempern wurden die Polymerproben in einem BERGHOF HR-500 Hochdruck-Autoklaven platziert und mit 50 bar $CO_2$ bei Raumtemperatur für 24 Stunden gesättigt.

Nach der Druckentlastung wurden die Proben für 18 min an der Luft belassen, um eine $CO_2$-Sättigung von ca. 6,5 % zu erreichen. Anschließend wurden die Proben für 15 Sekunden in ein heißes Silikonölbad bei 130 °C getaucht, um das Aufschäumen zu induzieren. Zur Stabilisierung der Zellen wurden die entstehenden Schaumstoffe zuerst in einem kalten Ölbad und danach in einem kalten Wasserbad für jeweils etwa 20 Sekunden abgekühlt. Zuletzt wurden die resultierenden Schaumstoffe in Seifenwasser gewaschen und vor einer weiteren Analyse für 12 Stunden an Luft getrocknet.

c) Schaumextrusion

Die Schaumextrusionen wurden auf einer Tandem-Extrusionslinie (Dr. Collin GmbH) durchgeführt (Doppelschneckenextruder mit 25 mm Schnecke und UD 42; Einschnecken-extruder mit 45 mm Schnecke und UD 30), die mit einer Schlitzdüse mit einem 0,6 mm Spalt und 3 mm Breite ausgestattet ist.

Es wurden extrudierter reiner XPS-Schaum sowie mehrere XPS-Schäume mit jeweils drei ausgewählten Additiv-Konzentrationen, nämlich 0,1 und 0,2 und 0,5 Gew.-% hergestellt und untersucht. Analoge Versuche werden mit SAN-Schäumen durchgeführt.

Die verschiedenen Additiv-Konzentrationen im Polymer wurden mittels eines gravimetrischen Dosierers durch Verdünnung eines Masterbatches mit reinem Polymer-Granulat mittels Kontrolle der Flussraten erhalten.

Eine Kombination von 4 Gew.-% $CO_2$ und 3 Gew.-% Ethanol wurde als (physikalisches) Treibmittel eingesetzt. Um eine XPS Referenz zu erhalten, wurde PS Granulat ohne Carbonsäureamid-Additiv auf dieselbe Weise extrudiert.

Die relevanten Verfahrensparameter für die Schaumextrusion sind in Tabelle 2 zusammengefasst.

**Tabelle 2**: Verfahrensparameter für die Schaumextrusion von PS Schaumstoffen

| Einlass-Schmelztemperatur [°C] | Auslass-Schmelztemperatur [°C] | Düsentemperatur [°C] | Schneckendrehzahl [rpm] | Durchsatz [kg/h] | Treibmittel $CO_2$ | [Gew.-%] EtOH |
|---|---|---|---|---|---|---|
| 260 | 106 - 130 | 123 - 132 | 8 | 4.5 | 4 | 3 |

In Tabelle 3 werden die Ergebnisse verschiedener, hergestellter Extrusionsschäume dargestellt, welche auf dem o.g. Polystyrol basieren. Die Extrusions-Schäume unterscheiden sich durch die eingesetzten Carbonsäureamid-Additive (jeweils 0,1 Gew.-%).

**Tabelle 3:** Mittlere Zellgröße und Schaumdichte von PS-Extrusionsschäumen ohne Additiv, mit 0,1 oder 0,5 Gew.-% IrgaclearXT 386, mit 0,1, 0,2 oder 0,5 Gew.-% Carbonsäureamid 3b

| **Additiv** | **Mittlere Zellgröße und Standardabweichung (Mikrometer)** | **Schaumdichte (kg/m$^3$)** |
|---|---|---|
| Kein Additiv | 632,1 +/- 183,9 | 52,3 |
| Irgaclear XT 386 (0,1 Gew.-%) | 25,7 +/- 7,8 | 72,6 |
| Irgaclear XT 386 (0,5 Gew.-%) | 31,3 +/- 10,1 | - |
| Bisamid (3b) (0,1 Gew.-%) | 14,4 +/- 4,7 | 76,5 |
| Bisamid (3b) (0,2 Gew.-%) | 14,7 +/- 5,5 | 82,8 |
| Bisamid (3b) (0,5 Gew.-%) | 10,7 +/- 4,3 | 71,2 |

Der in Tabelle 3 gezeigte Polystyrol-Schaum mit Carbonsäureamid (3b) zeigt bereits bei Verwendung von 0,1 Gew.-% des Additivs eine kleine Zellengröße und folglich eine signifikante Erhöhung der Zellenzahl. Es werden kleinzellige, geschlossen-zellige Schäume erhalten.

Bei Einsatz von 0,2 Gew.-% von Carbonsäurebisamid (3b) im Polystyrol beträgt die mittlere Zellengröße des Schaums 14,7 und die Schaumdichte 82,8 kg/m$^3$. Bei Einsatz von 0,5 Gew.-% von Carbonsäurebisamid (3b) im Polystyrol beträgt die mittlere Zellengröße des Schaums 10,7 Mikrometer und die Schaumdichte 71,2 kg/m$^3$.

Auch mit den anderen erfindungsgemäßen Bisamid-Derivaten (1a-d), (2a-d) und (3a, cd) wurden im Batchschaum-Verfahren bei den getesteten Konzentrationen (0,1; 0,25; 0,5 Gew.-% des Bisamids) in Polystyrol jeweils kleinzellige und geschlossen-zellige Schäume erhalten, die zudem eine sehr weitgehend homogene Zellengröße aufweisen.

Beim Einsatz des bekannten Trisamid-Derivats Irgaclear XT 386 im Batchschaum-Verfahren wurden hingegen bei den getesteten Konzentrationen (0,1; 0,25; 0,5 Gew.-% des Trisamids) in Polystyrol jeweils deutlich größer-zellige Schäume erhalten, die zudem keine homogene Zellengröße aufwiesen.

Im Schaumextrusions-Verfahren bei einem Einsatz von 0,1 Gew.-% an Irgaclear XT 386 im Polystyrol betrug die mittlere Zellengröße des Schaums 25,7 +/- 7,8 Mikrometer, beim Einsatz von 0,5 Gew.-% im Polystyrol betrug die mittlere Zellengröße des Schaums 31,3 Mikrometer.

Zudem wurden ausführliche Untersuchungen der Wärmeleitfähigkeit der XPS-Schäume (an Rundplättchen mit 60 mm Durchmesser) durchgeführt. Es wurde festgestellt, dass die Bisamide der Formel (I) selbst in geringer Konzentration im Polymer eingesetzt zu deutlich besseren Isolations-Eigenschaften (z.B. bei 0,1 Gew.% des Bisamids 3b im o.g. Polystyrol von + 7 %) bei den Schäumen führen als bei entsprechenden Schäume, hergestellt mit dem Trisamid-Additiv Irgaclear XT 386 in Polystyrol.

In weiteren Untersuchungen mit dem Batchschaum-Verfahren konnte gezeigt werden, dass die erfindungsgemäßen Additive auch in anderen Polymeren vorteilhaft eingesetzt werden können. So konnte mit dem Bisamid-Derivat (wie z.B. 3b) bereits bei Verwendung von 0,1 Gew.-% eine signifikante Reduzierung der mittleren Zellengröße in einem Styrol-Acrylnitril-Copolymer (Luran® 25100, INEOS Styrolution) erzielt werden kann.

Der mit Kohlenstoffdioxid (130°C, 25 Sekunden) erhaltene Polymerschaum hatte eine mittleren Zellendurchmesser des Schaums 16,4 +/- 6,1 Mikrometer und die Schaumdichte betrug 39,9 kg/m$^3$. Eine deutliche Erhöhung der Zellenzahl wurde festgestellt, es wurde ein kleinzelliger, geschlossen-zelliger SAN-Schaum erhalten. Auch war die Zellgrößenverteilung homogen.

Somit ermöglichen die Verbindungen der Formel (I) die Bereitstellung neuer geschlossen-zelliger Schaumprodukte, die sich auch in Verbundkörpern (mit mehreren Schichten) vorteilhaft einsetzen lassen.

## Patentansprüche

1. Verfahren zur Herstellung eines Schaumstoffs aus mindestens einer Styrol-Polymer-Komponente (S) und mindestens einem Additiv der allgemeinen Formel (I), umfassend die Schritte:

a. Erwärmen mindestens einer Styrol-Polymer-Komponente (S), um eine geschmolzene, polymere Formmasse zu erhalten,
b. Einbringen eines Treibmittels (T) in die geschmolzene, polymere Formmasse zur Ausbildung einer schäumbaren Zusammensetzung (Z), und
c. Aufschäumen der schäumbaren Zusammensetzung (Z), um einen geschäumten Formkörper zu erhalten,
wobei in der geschmolzenen polymeren Formmasse mindestens ein Carbonsäurebisamid der allgemeinen Formel (I) eingesetzt wird,

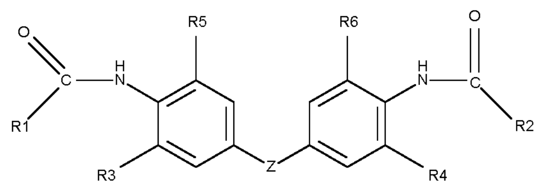
(I)

wobei bedeuten:

Z eine $C_1$-$C_5$-Alkylengruppe, insbesondere $-CH_2-$, oder ein Sauerstoffatom oder ein Schwefelatom,
R1 und R2 unabhängig voneinander einen verzweigten $C_3$-$C_{12}$-Alkylrest oder unverzweigten $C_1$-$C_{12}$-Alkylrest, einen $C_3$-$C_{12}$-Cycloalkylrest, oder einen Benzylrest;
R3, R4, R5 und R6 jeweils unabhängig voneinander Wasserstoff, einen unverzweigten $C_1$-$C_6$-Alkylrest oder einen verzweigten $C_3$-$C_6$-Alkylrest.

**2.** Verfahren nach Anspruch 1, **dadurch gekennzeichnet, dass** in der allgemeinen Formel (I) R1 und R2 unabhängig voneinander einen $C_3$-$C_{12}$-Cycloalkylrest, insbesondere einen Cyclohexylrest oder Cyclopentylrest, einen Butylrest oder einen Benzylrest bedeuten.

**3.** Verfahren nach Anspruch 1 oder 2, **dadurch gekennzeichnet, dass** in der allgemeinen Formel (I) Z eine Methylengruppe ist, und R3, R4, R5 und R6 jeweils unabhängig voneinander einen $C_1$-$C_6$-Alkylrest, insbesondere einen $C_1$-$C_2$-Alkylrest bedeuten.

**4.** Verfahren nach mindestens einem der Ansprüche 1 bis 3, **dadurch gekennzeichnet, dass** das Carbonsäurebisamid der allgemeinen Formel (I) in einer Menge von 0,01 bis 2,0 Gew.-%, bezogen auf das Gesamtgewicht der polymeren Formmasse eingesetzt wird.

**5.** Verfahren nach mindestens einem der Ansprüche 1 bis 4, **dadurch gekennzeichnet, dass** in die geschmolzene, polymere Formmasse ein Treibmittel (T) aus der Gruppe aus Pentan, Cyclopentan, Kohlenstoffdioxid und Ethanol oder einer Mischung eingebracht wird.

**6.** Verfahren nach mindestens einem der Ansprüche 1 bis 5, **dadurch gekennzeichnet, dass** als Styrol-Polymer-Komponente (S) ein Polystyrol (PS) und/oder ein Copolymer enthaltend Styrol und Acrylnitril, insbesondere Styrol-Acrylnitril (SAN), eingesetzt wird.

**7.** Schaumstoff, erhältlich durch ein Verfahren gemäß mindestens einem der Ansprüche 1 bis 6.

**8.** Schaumstoff gemäß Anspruch 7, der eine Dichte im Bereich von mindestens 30 $kg/m^3$ aufweist und der eine zumindest 50 % geschlossen-zellige Struktur aufweist.

**9.** Schaumstoff gemäß Anspruch 7 oder 8, der eine Dichte im Bereich von 45-85 $kg/m^3$ aufweist und der eine zumindest 90 % geschlossen-zellige Struktur aufweist.

**10.** Schaumstoff gemäß mindestens einem der Ansprüche 7 bis 9, dessen mittlerer Zellendurchmesser (D) von 0,1-25,0 Mikrometer beträgt, wobei der mittlere Zellendurchmesser (D) durch das in der Anmeldung offenbarte Verfahren bestimmt wird.

**11.** Schaumstoff gemäß einem der Ansprüche 7 bis 10, dessen mittlerer Zellendurchmesser (D) von 1,0- 16,0, insbesondere 3,0 bis kleiner als 15,0 Mikrometer beträgt, wobei der mittlere Zellendurchmesser (D) durch das in der

Anmeldung offenbarte Verfahren bestimmt wird.

**12.** Schaumstoff gemäß mindestens einem der Ansprüche 7 bis 11, wobei es sich um einen Polystyrol-basierten Schaumstoff handelt.

**13.** Schaumstoff gemäß mindestens einem der Ansprüche 7 bis 12, wobei es sich um einen Styrol-Acrylnitril-Copolymer-basierten Schaumstoff handelt.

**14.** Polymerzusammensetzung für die Herstellung eines Schaumstoffs enthaltend mindestens eine Styrol-Polymer-Komponente (S) und mindestens ein Carbonsäurebisamid der allgemeinen Formel (I) sowie ggf. weitere Additive,

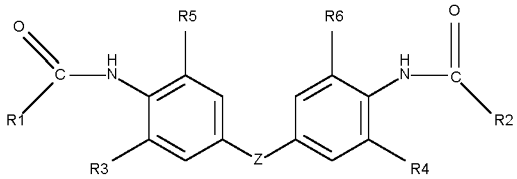

(I)

wobei bedeuten:

Z eine $C_1$-$C_5$-Alkylengruppe, insbesondere -$CH_2$-, oder ein Sauerstoff- oder Schwefelatom ;
R1 und R2 unabhängig voneinander einen verzweigten $C_3$-$C_{12}$-Alkylrest oder unverzweigten $C_1$-$C_{12}$-Alkylrest, einen $C_3$-$C_{12}$-Cycloalkylrest, oder einen Benzylrest;
R3, R4, R5 und R6 jeweils unabhängig voneinander Wasserstoff, einen unverzweigten $C_1$-$C_6$-Alkylrest oder einen verzweigten $C_3$-$C_6$-Alkylrest.

**15.** Verwendung eines Carbonsäurebisamids der allgemeinen Formel (I), definiert gemäß einem der Ansprüche 1 bis 3,

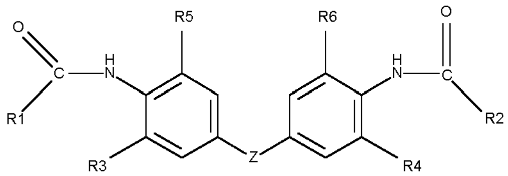

(I)

als ein den mittleren Zellendurchmesser (D) eines Schaums verkleinerndes Additiv bei der Herstellung von Schaumstoffen aus mindestens einem polymeren Material, insbesondere mindestens einer Styrol-Polymer-Komponente (S).

## Claims

**1.** Process for producing a foam from at least one styrene polymer component (S) and at least one additive of the general formula (I), comprising the steps of:

a. heating at least one styrene polymer component (S) to give a melted polymeric molding compound,
b. introducing a blowing agent (T) into the melted polymeric molding compound, to form a foamable composition (Z), and
c. foaming the foamable composition (Z) to give a foamed molding,
wherein at least one carboxylic bisamide of the general formula (I) is used in the melted polymeric molding compound,

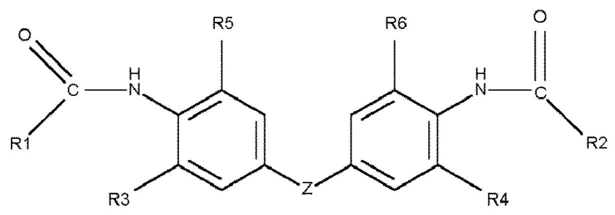

(I)

wherein:

Z is a $C_1$-$C_5$ alkylene group, more particularly $-CH_2-$, or an oxygen atom or a sulfur atom,
R1 and R2 independently of one another are a branched $C_3$-$C_{12}$ alkyl rest or unbranched $C_1$-$C_{12}$ alkyl rest, a $C_3$-$C_{12}$ cycloalkyl rest, or a benzyl rest;
R3, R4, R5 and R6 each independently of one another are hydrogen, an unbranched $C_1$-$C_6$ alkyl rest or a branched $C_3$-$C_6$ alkyl rest.

2. Process as claimed in claim 1, **characterized in that** in the general formula (I) R1 and R2 independently of one another are a $C_3$-$C_{12}$ cycloalkyl rest, more particularly a cyclohexyl rest or cyclopentyl rest, a butyl rest or a benzyl rest.

3. Process as claimed in claim 1 or 2, **characterized in that** in the general formula (I) Z is a methylene group and R3, R4, R5 and R6 each independently of one another are a $C_1$-$C_6$ alkyl rest, more particularly a $C_1$-$C_2$ alkyl rest.

4. Process as claimed in at least one of claims 1 to 3, **characterized in that** the carboxylic bisamide of the general formula (I) is used in an amount of 0.01 to 2.0 wt%, based on the total weight of the polymeric molding compound.

5. Process as claimed in at least one of claims 1 to 4, **characterized in that** a blowing agent (T) from the group of pentane, cyclopentane, carbon dioxide and ethanol or a mixture is introduced into the melted polymeric molding compound.

6. Process as claimed in at least one of claims 1 to 5, **characterized in that** the styrene polymer component (S) used is a polystyrene (PS) and/or a copolymer containing styrene and acrylonitrile, more particularly styrene-acrylonitrile (SAN).

7. Foam obtainable by a process as claimed in at least one of claims 1 to 6.

8. Foam as claimed in claim 7, having a density in the region of at least 30 kg/$m^3$ and having an at least 50% closed-cell structure.

9. Foam as claimed in claim 7 or 8, having a density in the range of 45-85 kg/$m^3$ and having an at least 90% closed-cell structure.

10. Foam as claimed in at least one of claims 7 to 9, having a mean cell diameter (D) of 0.1-25.0 micrometers, wherein the mean cell diameter (D) is determined according to the method disclosed in the application.

11. Foam as claimed in any of claims 7 to 10, having a mean cell diameter (D) of 1.0-16.0, more particularly 3.0 to less than 15.0 micrometers, wherein the mean cell diameter (D) is determined according to the method disclosed in the application.

12. Foam as claimed in at least one of claims 7 to 11, which is a polystyrene-based foam.

13. Foam as claimed in at least one of claims 7 to 12, which is a styrene-acrylonitrile copolymer-based foam.

14. Polymer composition for producing a foam, comprising at least one styrene polymer component (S) and at least one carboxylic bisamide of the general formula (I) and also optionally further additives,

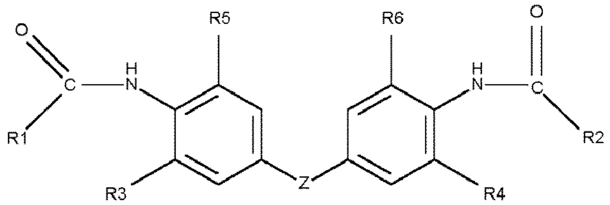

(I)

wherein:

Z is a $C_1$-$C_5$ alkylene group, more particularly $-CH_2-$, or an oxygen atom or a sulfur atom;
R1 and R2 independently of one another are a branched $C_3$-$C_{12}$ alkyl rest or unbranched $C_1$-$C_{12}$ alkyl rest, a $C_3$-$C_{12}$ cycloalkyl rest, or a benzyl rest;
R3, R4, R5 and R6 each independently of one another are hydrogen, an unbranched $C_1$-$C_6$ alkyl rest or a branched $C_3$-$C_6$ alkyl rest.

**15.** Use of a carboxylic bisamide of the general formula (I), defined as claimed in any of claims 1 to 3,

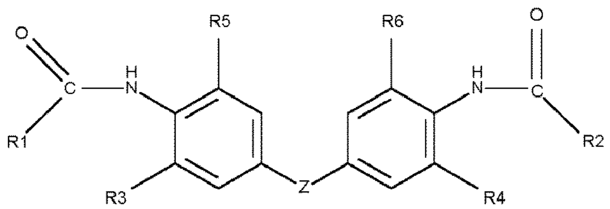

(I)

as an additive reducing the mean cell diameter (D) of a foam in the production of foams from at least one polymeric material, more particularly at least one styrene polymer component (S).

**Revendications**

**1.** Procédé de préparation d'une mousse à partir d'au moins un composant polymère de styrène (S) et d'au moins un additif de formule générale (I), comprenant les étapes :

a. chauffage d'au moins un composant polymère de styrène (S), afin d'obtenir une masse de moulage polymère fondue,
b. introduction d'un agent gonflant (T) dans la masse de moulage polymère fondue afin de former une composition pouvant être moussée (Z) et
c. moussage de la composition pouvant être moussée (Z), afin d'obtenir un corps moulé moussé,

au moins un bisamide d'acide carboxylique de formule générale (I) étant utilisé dans la masse de moulage polymère fondue,

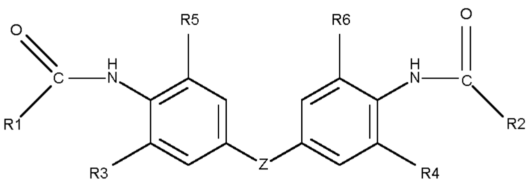

(I)

dans laquelle

Z signifie un groupe $C_1$-$C_5$-alkylène, en particulier $-CH_2-$, ou un atome d'oxygène ou un atome de soufre,
R1 et R2 signifient, indépendamment l'un de l'autre, un radical $C_3$-$C_{12}$-alkyle ramifié ou un radical $C_1$-$C_{12}$-alkyle

non ramifié, un radical $C_3$-$C_{12}$-cycloalkyle ou un radical benzyle ;
R3, R4, R5 et R6 signifient, à chaque fois indépendamment l'un de l'autre, hydrogène, un radical $C_1$-$C_6$-alkyle non ramifié ou un radical $C_3$-$C_6$-alkyle ramifié.

2. Procédé selon la revendication 1, **caractérisé en ce que**, dans la formule générale (I), R1 et R2 signifient, indépendamment l'un de l'autre, un radical $C_3$-$C_{12}$-cycloalkyle, en particulier un radical cyclohexyle ou cyclopentyle, un radical butyle ou un radical benzyle.

3. Procédé selon la revendication 1 ou 2, **caractérisé en ce que**, dans la formule générale (I), Z représente un groupe méthylène et R3, R4, R5 et R6 signifient, à chaque fois indépendamment les uns des autres, un radical $C_1$-$C_6$-alkyle, en particulier un radical $C_1$-$C_2$-alkyle.

4. Procédé selon au moins l'une des revendications 1 à 3, **caractérisé en ce que** le bisamide d'acide carboxylique de formule générale (I) est utilisé en une quantité de 0,01 à 2,0% en poids, par rapport au poids total de la masse de moulage polymère.

5. Procédé selon au moins l'une des revendications 1 à 4, **caractérisé en ce qu'**un agent gonflant (T) du groupe formé par le pentane, le cyclopentane, le dioxyde de carbone et l'éthanol ou un mélange est introduit dans la masse de moulage polymère fondue.

6. Procédé selon au moins l'une des revendications 1 à 5, **caractérisé en ce qu'**on utilise, comme composant polymère de styrène (S), un polystyrène (PS) et/ou un copolymère contenant du styrène et de l'acrylonitrile, en particulier le styrène-acrylonitrile (SAN).

7. Mousse, pouvant être obtenue par un procédé selon au moins l'une des revendications 1 à 6.

8. Mousse selon la revendication 7 qui présente une densité dans la plage d'au moins 30 $kg/m^3$ et qui présente au moins à raison de 50% une structure à cellules fermées.

9. Mousse selon la revendication 7 ou 8, qui présente une densité dans la plage de 45-85 $kg/m^3$ et qui présente au moins à raison de 90% une structure à cellules fermées.

10. Mousse selon au moins l'une des revendications 7 à 9, dont le diamètre moyen de cellule (D) est de 0,1-25,0 micromètres, le diamètre moyen de cellule (D) étant déterminé par le procédé divulgué dans la demande.

11. Mousse selon au moins l'une des revendications 7 à 10, dont le diamètre moyen de cellule (D) est de 1,0-16,0, en particulier de 3,0 à moins de 15,0 micromètres, le diamètre moyen de cellule (D) étant déterminé par le procédé divulgué dans la demande.

12. Mousse selon au moins l'une des revendications 7 à 11, la mousse étant une mousse à base de polystyrène.

13. Mousse selon au moins l'une des revendications 7 à 12, la mousse étant une mousse à base de copolymère de styrène-acrylonitrile.

14. Composition polymère pour la préparation d'une mousse contenant au moins un composant polymère de styrène (S) et au moins un bisamide d'acide carboxylique de formule générale (I) ainsi que le cas échéant d'autres additifs

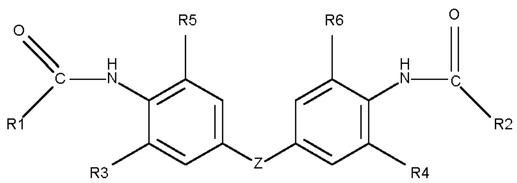

(I)

dans laquelle

Z signifie un groupe $C_1$-$C_5$-alkylène, en particulier $-CH_2-$, ou un atome d'oxygène ou un atome de soufre ;

R1 et R2 signifient, indépendamment l'un de l'autre, un radical $C_3$-$C_{12}$-alkyle ramifié ou un radical $C_1$-$C_{12}$-alkyle non ramifié, un radical $C_3$-$C_{12}$-cycloalkyle ou un radical benzyle ;
R3, R4, R5 et R6 signifient, à chaque fois indépendamment l'un de l'autre, hydrogène, un radical $C_1$-$C_6$-alkyle non ramifié ou un radical $C_3$-$C_6$-alkyle ramifié.

**15.** Utilisation d'un bisamide d'acide carboxylique de formule générale (I) définie selon l'une des revendications 1 à 3,

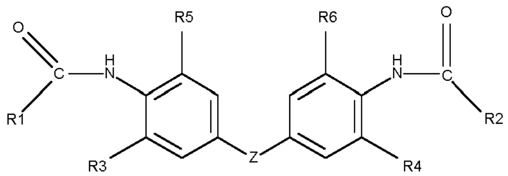

(I)

en tant qu'additif diminuant le diamètre moyen de cellule (D) d'une mousse lors de la préparation de mousses à partir d'au moins un matériau polymère, en particulier à partir d'au moins un composant polymère de styrène (S).

FIG. 1

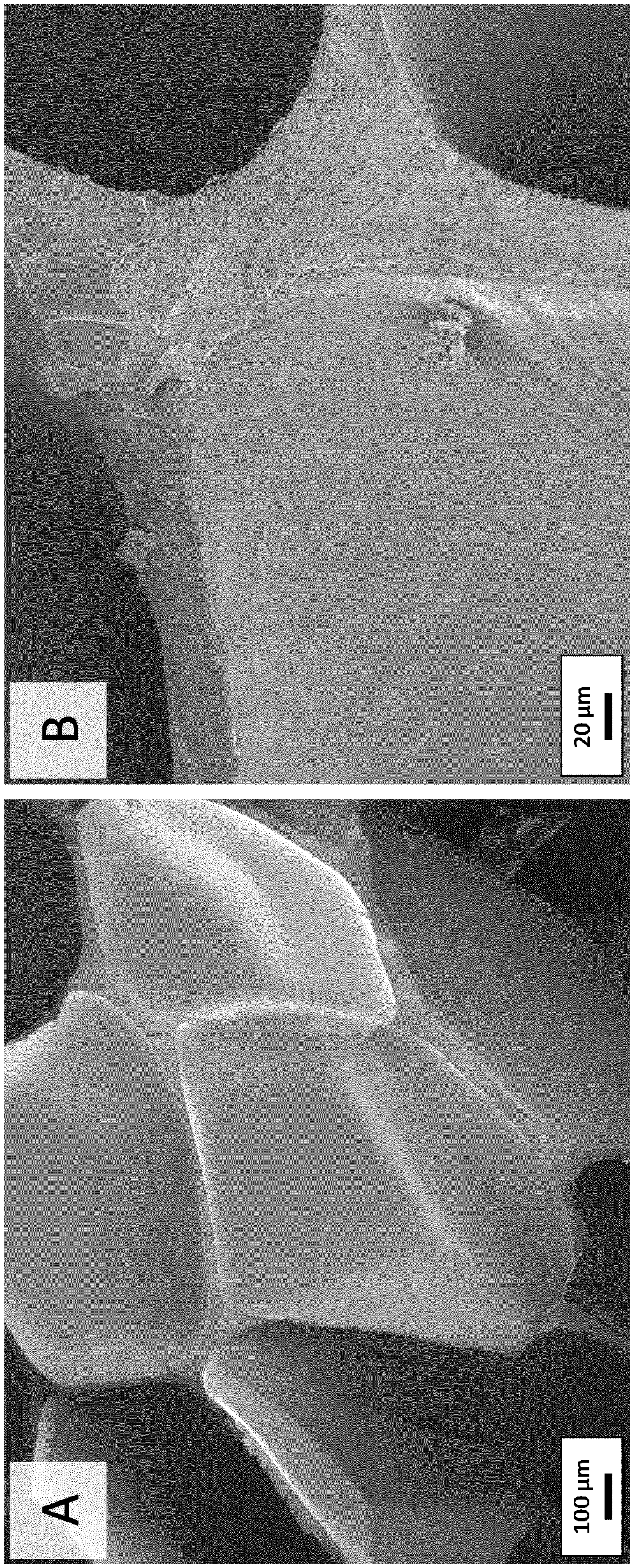

FIG. 2

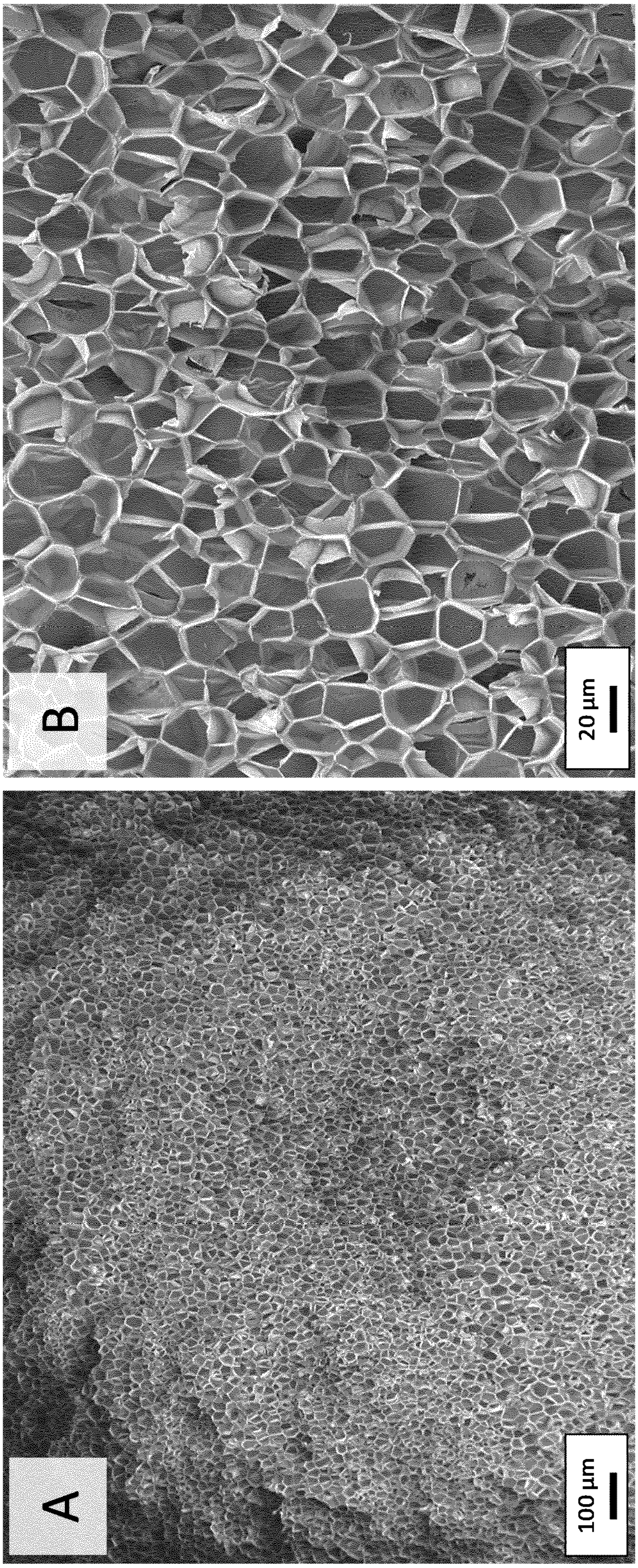

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 681522 A, Dow
- US 5210105 A, Dow
- EP 1031600 A
- EP 1385902 B
- WO 2004072168 A
- EP 1661939 A
- US 8420721 B
- DE 102011083434 A
- WO 2015090509 A
- EP 3083802 B
- WO 2015197152 A


### In der Beschreibung aufgeführte Nicht-Patentliteratur


- **M. STUMPF et al.** *Journal of Cellular Plastics,* 2011, vol. 47 (6), 519-534
- **M. MÖRL et al.** *Journal of Cellular Plastics,* 2018, vol. 54, 483-498
- **M. AKSIT ; B. KLOSE et al.** *Journal of Cellular Plastics,* 2019, vol. 55 (3), 249-261
- **M. AKSIT ; C. ZHAO et al.** *Polymers,* 2019, vol. 11, 268